# EUROPEAN PATENT APPLICATION

(11) **EP 4 183 871 A1**
(43) Date of publication of application: **24.05.2023**
(21) Application number: 21306627.7
(22) Date of filing: 23.11.2021
(51) Int. Cl.: C12N 5/0783, A61K 35/17

(54) **PROCESS FOR PREPARING A COMPOSITION COMPRISING A COMBINED CELL POPULATION**

(71) Applicant: Université d'Aix-Marseille, 13007 Marseille (FR); Centre national de la recherche scientifique, 75016 Paris (FR); Institut National de la Santé et de la Recherche Médicale (INSERM), 75013 Paris 13 (FR); Institut Paoli Calmettes, 13009 Marseille (FR)
(72) Inventor: OLIVE, Daniel, 13009 MARSEILLE (FR); DEVILLIER, Raynier, 13010 MARSEILLE (FR); SALEM, Nassim, 13005 MARSEILLE (FR)
(74) Representative: IPAZ

(57) **Abstract**

The present invention pertains to a process for preparing a composition comprising a combined cell population comprising at least 90% Natural Killer (NK) cells and gamma-delta T (γδT) lymphocytes. The present invention also pertains to said composition comprising a combined cell population comprising NK cells and γδT lymphocytes and its use in medicine.

## Description

### FIELD OF THE INVENTION

The present invention pertains to a process for preparing a composition comprising a combined cell population comprising Natural Killer (NK) cells and gamma-delta T (γδT) lymphocytes. The present invention also pertains to said composition comprising a combined cell population comprising NK cells and γδT lymphocytes and its use in medicine. The present invention therefore pertains to the technical domain of processes for preparing cell compositions, cells composition and their medical application, in particular for immunotherapy.

### TECHNICAL BACKGROUND

Within its use in an allogeneic context, adoptive T lymphocytes immunotherapy is limited by the induction of graft-versus host disease (GVHD). Indeed, donor allogeneic T cells can recognize patient healthy tissues though peptide presentation by HLA class 1 molecules, leading to donor alloreactive T cell activation, cytokine production and proliferation. The use of donor lymphocyte infusion (DLI) after allogeneic hematopoietic stem cell transplantation (allo-SCT) is a striking example of this statement. Although DLI can improve graft-versus-tumor (GVT) effect after allo-SCT, 20 to 40% of patients presented with DLI suffered from GVHD. Thus, immunotherapeutic alternatives should be developed aiming provide strong GVT effect without inducing GVHD.

NK belong to the group of T lymphocytes and represent an interesting alternative because of their intrinsic biological properties of target cell recognition based on integrated signals coming from both activating and inhibitory receptors rather than peptide presentation though HLA class I molecules. It was previously demonstrated that: 1) NK cell have strong antitumor effect; 2) allogeneic NK cells do not induce GVHD; 3) adoptive allogeneic NK cell therapy is feasible both after allo-SCT and outside the context of allo-SCT. A donor-derived activated NK cell infusion after all-SCT is feasible and safe.

Among T lymphocytes, gamma delta T lymphocytes, which also designated as "γδT lymphocytes" or "γδT cells", represent a minor subset of cells within peripheral blood in humans and harbor biological properties of both innate and adaptive immunity. In particular, they share some activating and inhibitory receptor with NK cells and provide a strong antitumor effect without triggering GVHD.

WO 2016/005752 "*γδT cells and their use"* discloses a selective expansion process for preparing γδT lymphocytes for the allogeneic or autologous treatment of subjects. Said process comprises isolating PBMCs from a first subject, contacting said cells with zoledronic acid and with IL-2, culturing said mixture for at least 5 days and recovering a cell population comprising more than 80 % of cells that are T lymphocytes, wherein at least 60 % of the total population of T lymphocytes are γδT lymphocytes, and less than 25 % of the total population of T lymphocytes are NK cells.

Kabeltz et al ("Cancer immunotherapy with γδT cells: many paths ahead of us", Cell. And Mol. Immunology, 2020, 17:925-39) discloses a protocol for expanding VδT cells comprising contacting PBMCs with zoledronic acid and IL-2.

Sebestyen et al ("Translating γδT cells and their receptors into cancer cells therapies" Nature Reviews, Drug Discovery, 2019) discloses immunotherapeutic methods using γδT cells.

WO 2006/017954 *"Preparation of human γδT cells presenting antigens and their use in immunotherapy"* discloses processes for generating γδT cells from T cells isolated from PBMCs, either by an initial step of selecting T lymphocytes on Percoll then expanding γδT cells, or by positively selecting γδT from PBMCs.

WO 2015/061694 *"Polyclonal γδT cells for immunotherapy"* discloses a process for generating γδT cells, said process comprising a step of depleting CD56+ NK cells before expanding said T cells.

WO 2012/156958 "*T lymphocytes cell line comprising γδT cells"* discloses a process for generating γδT cells comprising a step of positive or negative selection of said γδT cells f.

The inventors have now designed a combined expansion process allowing to concomitantly expand NK and γδT cells from a sample from a first subject, said process provides a cell composition wherein:
- at least 90% of the cells are NK cells and γδT lymphocytes and
- less 5% of the cells are αβT lymphocytes.

A process according to the invention allows the generation, in a limited number of easy steps, of a useful cell composition. More particularly, a step of cell sorting before cell expansion is only optional. The number of cells of interest, consisting of combined NK cells and γδT lymphocytes, is higher than the number of NK cells and γδT lymphocytes obtained via independent culture processes of prior art.

The characterization of the cell population obtained by a process according to the present invention demonstrates that:
- the cell population expresses surface markers allowing their sorting and quantification
- in appropriate stimulating conditions, the cell population synthesize normal amounts of cytokines and transducers
- the cells are compatible with the usual genetic modification of cells for immunotherapy products, such as, for example, viral transduction,
- the cell population exerts an in vitro anti-tumor effect.

The combined presence, in a composition according to the invention, of both NK cells and γδT lymphocytes, allows a broader spectrum of antitumor properties than compositions comprising mainly NK cells or compositions comprising mainly γδT lymphocytes. For example, acute Myeloid Leukemia (AML) primary blasts can have differential sensitivity to NK and γδT cell lysis. In particular, AML blasts can be highly resistant to γδT cell specific lysis while highly sensitive to NK cell specific lysis or vice versa. A cell therapy product of the invention, combining both NK cells and γδT cells, therefore represents a simple and validated tool useful for overcoming resistance to cellular immunotherapy.

### SUMMARY OF THE INVENTION

In a first aspect, the present invention provides a process for generating a cell composition comprising NK cells and gamma-delta T (γδT) lymphocytes.

In a second aspect, the present invention provides a composition comprising a cell population comprising NK cells and gamma-delta T (γδT) lymphocytes. According to this second aspect, the present invention provides a cell composition comprising, as a major cellular component NK cells and gamma-delta T (γδT) lymphocytes, which corresponds to a percentage of cells being NK cells or gamma-delta T (γδT) lymphocytes of at least 90%, 91%, 92%, 93%, 94%, 95%, 96%, 97%, 98% or 99% of the total number of living cells of the composition. In a particular embodiment of a composition of the invention, at least 25 % of the total number of cells are NK cells and at least 20 % of the total number of cells are γδT lymphocytes.

In a third aspect, the present invention provides the use of a composition comprising a cell population comprising NK cells and gamma-delta T (γδT) lymphocytes in medicine, for cellular therapy, alone or in combination with a drug or treatment such as, for example a chemotherapeutic drug, a cytokine or any other appropriate prophylactic or therapeutic product.

Characteristics, advantages and uses of the subject-matter of the present invention are more detailed hereunder, in an illustrated and non-limiting way. When present, the disclosure of the ranges expressed as "from ... to ..." mean that the limits are included in said ranges.

### DETAILED DESCRIPTION OF THE INVENTION

In a first aspect, the present invention relates to a process for preparing a cell composition comprising NK cells and γδT lymphocytes, said process comprising the steps of:
a) contacting a biological sample isolated from a first subject, said sample comprising NK cells and γδT lymphocytes, with feeder cells treated with a phosphoantigen stimulating agent,
b) contacting said sample and said feeder cells from step a) with a growth media comprising at least one phosphoantigen stimulating agent and at least one interleukin, for at least 5 days,
c) isolating the cell composition obtained from step b), wherein said cell composition comprises:
   - at least 90% of NK cells and γδT lymphocytes and
   - less than 5% of αβT lymphocytes.

In a particular embodiment, the present invention relates to a process for preparing a cell composition comprising NK cells and γδT lymphocytes, wherein said cell composition comprises:
- at least 90% of NK cells and γδT lymphocytes,
- less than 5% of αβT lymphocytes,
- wherein at least 25% of the living cells are NK cells.

According to the present invention, "said cell composition comprises at least 90% of NK cells and γδT lymphocytes" means that, among the total number of living cells present in the cell composition, at least 90% of said cells are characterized as NK cells or as γδT lymphocytes, according to characterization methods known in the art.

Also, by "said cell composition comprises less than 5% of αβT lymphocytes" it is meant that, among the total number of living cells present in the cell composition, at most 5% of said cells are characterized as αβT lymphocytes, according to methods known in the art.

Said methods to characterize a cell population include, but are not restricted to, the characterization of the presence of known cellular surface determinants.

The presence or absence of a particular determinant on the cell surface are detected by technical tools and methods well known by a person skilled in the art. Said tools and methods include, for example, publicly available labelled monoclonal antibodies and flow cytometry detection by Fluorescence Activated Cells Sorting (FACS).

T lymphocytes are cells characterized by their surface expression of CD3 and of T cells receptors (TCR). A majority of T lymphocytes express the αβ type of T-cell receptor (αβTCR) and said cells are designated as αβT cells, or "conventional" T cells.

Cytotoxic T lymphocytes are defined as αβT lymphocytes expressing the CD3 and CD8 surface determinants. Helper T lymphocytes are defined as αβT lymphocytes expressing the CD3 and CD4 surface determinants.

Gamma delta T cells (γδT cells) are T lymphocytes that express a unique type of T-cell receptor (TCR), said TCR being composed of one γ-chain and one δ-chain. Gamma delta T cells are characterized by the presence, on their surface, of γδ TCR and of CD3.

The group of γδ TCR includes, but is not limited to, TCR-Vδ1, TCR-Vδ2 and TCR-Vy9. γδT cells surface marker other than CD3 include, but are not limited to: CD4, CD8, CD69, CD56, CD27 and CD45A.

Gamma delta T cells are of low abundance in the body, are found in the gut mucosa, skin, lungs and uterus, and are involved in the initiation and propagation of immune responses. The major γδT lymphocytes subset in human beings is designated by Vγ9Vδ2.

NK cells are defined as T lymphocytes characterized by the presence on their surface of the 140 kD isoform of CD56, or Neural Cell Adhesion Molecule (NCAM), and by the absence of the CD3 determinant.

In a process according to the present invention, a sample comprising NK cells and γδT lymphocytes can be any biological sample comprising said cells, and may be chosen among: blood or umbilical cord blood, wherein blood is optionally depleted from a part of its cells, peripheral blood mononuclear cells (PBMCs), tissue sample and induced pluripotent stem cells (iPSC).

More particularly, in a process of the invention, a blood sample comprising NK cells and γδT lymphocytes can be total blood or blood depleted from at least one cell population, for example its red blood cells.

In a process of the invention, when said sample is PBMCs, isolated from blood or from apheresis or leukapheresis material, as known by a person skilled in art for example by density gradient centrifugation, said PBMCs can be total PBMCs or PBMCs depleted from at least a part of its αβT lymphocytes. Wherein said sample is a tissue sample, it can be for example a tumor sample obtained from biopsy or surgery, it may also be a sample obtained from a tumor micro-environment.

When appropriate, said sample can be subject to cryopreservation prior to subsequent expansion culture, or used as fresh material.

For a process of the invention, any growth media and feeder cells known by a person skilled in the art may be used. A typical example of feeder cells is K562 cells.

By "phosphoantigen stimulating agent" it is intended a compound which, when contacted with a cell, stimulates the cell surface expression of phosphorus-containing non peptide antigens. Therefore, in the presence of an effective concentration of a phosphoantigen stimulating agent, the level of cell surface expression of phosphorus-containing non peptide antigens is augmented when compared to their level in the absence of any phosphoantigen stimulating agent.

Said phosphoantigen stimulating agent can be chosen among:
- aminobiphosphonates such as: zoledronic acid, pamidronic acid and alendronic acid, their salt and/or their hydrate,
- phosphorylated intermediates from the isoprenoid biosynthesis pathway, such as isopentenyl-pyrophosphate (IPP) and (E)-4-hydroxy-3-methylbut-2-enyl pyrophosphate (HMBPP), bromohydrin pyrophosphate (BrHPP).
- inhibitors of farnesyldiphosphate synthase (FDPS),
and known derivatives thereof.

During step b) of a process of the invention, the concentration of said at least one phosphoantigen activating agent is of at least 0,05 µM, preferably from 0,5 µM to 100 µM, from 0,1 to 50 µM, from 1 to 10 µM, more preferably of 5 µM in the growth medium. In particular embodiments, the preferred concentration of zoledronic acid is from 0,1 to 10 µM, wherein the preferred concentration of pamidronic acid or alendronic acid is from 1 to 30 µM.

In a process according to the invention, any interleukin known by a person skilled in the art may be used. Preferably, said at least one interleukin is chosen among: IL-2, IL-21 and IL-15, and combinations thereof. When possible, a person skilled in the art will use a human and/or recombinant interleukin.

In the growth media, said at least one interleukin is present at a concentration from 50 to 2000 UI/ml, preferably from 400 to 1000 UI/ml.

In a process according to the invention, the γδT lymphocytes may be activated and proliferation from PBMCs via the addition, for example, of zoledronic acid at day 0 of step b) and via the addition of interleukin, such as IL-2, in a continuous way over at least 5 days of culture.

In a particular embodiment, the present invention relates to a process for preparing a cell composition comprising NK cells and γδT lymphocytes, said process comprising the steps of:
a) contacting a biological sample isolated from a first subject, said sample comprising NK cells and γδT lymphocytes, with feeder cells treated with a phosphoantigen stimulating agent,
b) contacting said sample and said feeder cells from step a) with a growth media comprising zoledronic acid and interleukin-2, for at least 5 days,
c) isolating the cell composition obtained from step b), wherein said cell composition comprises:
   - at least 90% of NK cells and γδT lymphocytes and
   - less than 5% of αβTlymphocytes.

A process of the invention comprises contacting said sample and said feeder cells from step a) with a growth media comprising at least one phosphoantigen stimulating agent and at least one interleukin, for an expansion step of at least 5 days, at least 6, 7, 8, 9, 10, 11, 12, 13 or 14 days. Culture is typically performed at a temperature comprised between 34 and 38°C, preferably at 37°C, in the presence of 2 to 10% CO₂, more preferably 5% CO₂. Culture medium may be added during the culture step.

After said expansion step, cells may be harvested and resuspended in an appropriate solution or buffer.

In another particular embodiment, a process according to the present invention comprises a step of depletion from αβT lymphocytes of said biological sample isolated from a first subject, prior to its contact with feeder cells treated with a phosphoantigen stimulating agent.

In another particular embodiment, a process according to the present invention comprises a step of depletion from T lymphocytes of said cell composition isolated at step c).

Depletion of T lymphocytes is performed by any method known by a person skilled in the art, it can be based on a negative selection or a positive selection methodology.

According to this first aspect, the present invention relates to a process for preparing a cell composition comprising NK cells and γδT lymphocytes from a biological sample from a first subject, for providing in an autogenic way a cell population comprising NK cells and γδT lymphocytes to said first subject.

The present invention also relates to a process for preparing a cell composition comprising NK cells and γδT lymphocytes from a biological sample from a first subject, for providing in an allogenic way a cell population comprising NK cells and γδT lymphocytes to a second subject.

Said first and/or second subject can be a vertebrate, for example a mammal, for example a human, or a research animal such as, for example, a horse, a cow, a goat, a mouse, a rabbit or a pig. In a particular embodiment, said first and second subjects are humans.

In a second aspect, the present invention relates to a cell composition comprising:
- at least 90% of NK cells and γδT lymphocytes
- less than 5% of αβT lymphocytes.
obtained by a process comprising the steps of:
a) contacting a biological sample isolated from a first subject, said sample comprising NK cells and γδT lymphocytes, with feeder cells treated with a phosphoantigen stimulating agent,
b) contacting said sample and said feeder cells from step a) with a growth media comprising zoledronic acid and interleukin-2, for at least 5 days,
c) isolating the cell composition obtained from step b).

In a particular embodiment of this second aspect, the present invention relates to a cell composition obtained by a process according to the invention, said cell composition comprising at least 90%, 91%, 92%, 93%, 94%, 95%, 96%, 97%, 98% or 99% of NK cells and γδT lymphocytes.

In a particular embodiment, the present invention relates to a cell composition obtained by a process according to the invention, said cell composition comprising at least 25%, 30%, 35%, 40%, 45%, 50%, 55%, 60%, 65%, 66%, 67%, 68%, 69%, 70%, 71%, 72%, 73%, 74% or 75% of NK cells.

In a particular embodiment, the present invention relates to a cell composition obtained by a process according to the invention, said cell composition comprising at least 10%, 15%, 20%, 21%, 25%, 30% or 35% of γδT lymphocytes. In another particular embodiment, the present invention relates to a cell composition obtained by a process according to the invention, said cell composition comprising at most 20%, 30%, 40%, or 50% of γδT lymphocytes.

In another particular embodiment, the present invention relates to a cell composition obtained by a process according to the invention, said in cell composition at least 80%, 85%, 90 or 95% of the total number of T lymphocytes are γδT lymphocytes.

In a particular embodiment, the present invention relates to a cell composition obtained by a process according to the invention, said cell composition comprising less than 5%, 4%, 3%, 2% or 1% of αβT lymphocytes.

In another particular embodiment, the present invention relates to a cell composition comprising at least 90% of NK cells and γδT lymphocytes, wherein, among the total number of cells in the composition:
- 75 +/-5% are NK cells and/or
- 25 +/-5% are γδT lymphocytes.

In a third aspect, the present invention relates to a cell composition according to the present invention, and to a cell composition obtained by a process according to the present invention, for its use in medicine. In this aspect, the number of NK cells and γδT lymphocytes are sufficient to exert a therapeutic effect to a recipient subject.

In another particular embodiment, the present invention relates to a cell composition according to the present invention or obtained by a process according to the present invention, for its use in medicine, wherein said biological sample is provided from a first subject and said cell composition is used for said first subject, for an autogenic use.

In another particular embodiment of this aspect, the present invention relates to a cell composition according to the present invention or obtained by a process according to the present invention, for its use in medicine wherein said biological sample is provided from a first subject and said cell composition is used for a second subject, for an allogenic use.

In another particular embodiment of this aspect, the present invention relates to a cell composition according to the present invention or obtained by a process according to the present invention, for its use in medicine simultaneously, separately or sequentially with a drug.

The present invention also relates to a cell composition according to the present invention or obtained by a process according to the present invention, for its use in medicine, for the prevention or the treatment of cancer or infection.

A cell composition according to the present invention or obtained by a process according to the present invention, may be used in medicine for the prevention or the treatment of a cancer such as haematological and/or solid tumors.

A cell composition according to the present invention or obtained by a process according to the present invention, may be used in medicine for the prevention or the treatment of a virus, fungi or protozoa infection.

In a fourth aspect, the present invention relates to a pharmaceutical composition comprising a cell composition obtained by a process according to the invention, or a cell composition according to the invention, and a pharmaceutically acceptable carrier.

The present invention further relates to a method of preventing or treating an infection or cancer in a subject comprising the step of providing said subject with a cell composition according to the present invention, or a cell composition obtained by a process according to the present invention.

Characteristics, advantages and uses of the subject-matter of the present invention are more detailed hereunder, in an illustrated and non-limiting way. When present, the disclosure of the ranges expressed as "from ... to ..." mean that the limits are included in said ranges.

Any one embodiment described herein can be combined with any one or more other embodiments of the invention, unless such combination is expressly disclaimed or improper. The invention is described with reference to particular examples, but it is understood by persons skilled in the art that various changes in the forms and details can be made without departing from the scope of the present invention.

### BRIEF DESCRIPTION OF THE FIGURE:

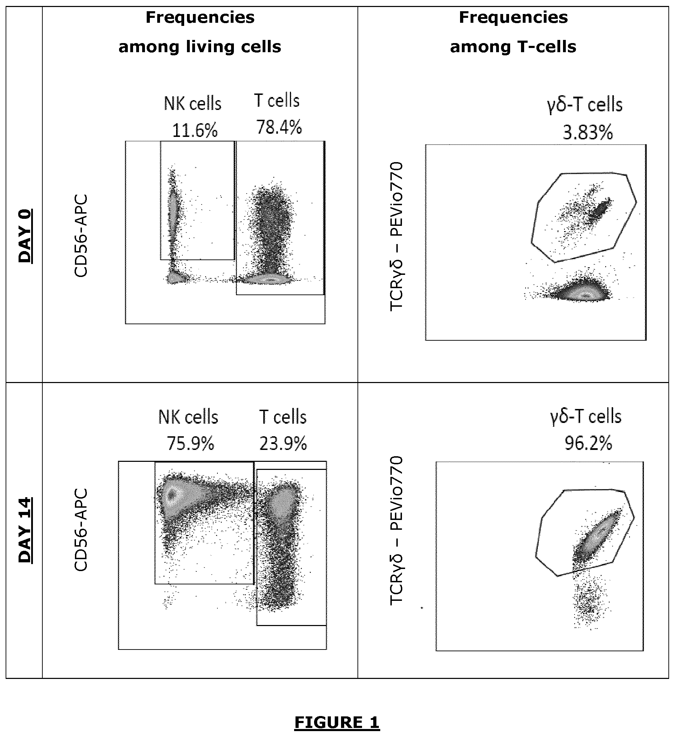

Figure 1 represents the flow cytometry plots showing the immunophenotyping of a representative cell population obtained after 14 days by a process according to the invention. The two upper panels represent the characterization of the cell population at day 0 of the cell culture, the two lower panels represent the characterization of the cell population at day 14 of the cell culture. The upper and lower left panels represent the frequencies of NK cells and of the total T cells among the total living cells, respectively. The upper and lower right panels represent the frequencies of γδT cells among the total living T cells, respectively.

### EXAMPLE

### Combined expansion of NK cells and γδT lymphocytes Material and methods

Donor PBMCs were obtained from gradient lymphocyte separation. Fresh or frozen/thawed PBMCs can be used for starting the expansion process, with similar results on fold expansion and final cell product composition.

For the feeder cell preparation, K562 feeder cells were cultured in RPMI 10% fetal calf serum (RPMI+10FCS) and pretreated with zoledronic acid (20 µM) overnight the day before expansion.

On Day 0 (beginning of the expansion): 10 million PBMCs were contacted with 10 million irradiated (100Gy) K562 feeder cells in a total volume of 20 mL of RPMI-10FCS in presence of zoledronic acid (5 µM) and IL-2 (500 UI/mL) in a T75 flask. Initial cell amount can be adapted to fit smaller or larger size culture flask, by keeping PBMC/feeder cell ratio.

Cells were subsequently manually or automatically counted and the frequencies of living NK, γδT and conventional αβT cells was determined by flow cytometry based on the expression of CD3, CD56, γδ-TCR and a viability marker, on days +5, +7, +9, +12 and +14. Fresh medium and IL-2 were added to keep cell concentration near 1 million per mL.

The antitumor functions of both NK and γδT cells were assessed by determining the induction of the expression of CD107a and the production of IFN-γ and TNF-α, in the presence of target cell lines: K562, 721.221, Daudi and HL60. Cells were co-cultivated for 4 hours at 1:1 effector:target ratio.

### Characterization of the cell composition

According to this first example, after 14-day culturing (N=5), the final cell product comprised a median of 1,270.10⁶ NK cells and 288.10⁶ γδT cells. This corresponds, respectively, to a 616 and 538 fold-increase for NK and γδT cells when compared to their initial presence. By contrast, the expansion of conventional αβT cells is very low, with a median fold increase of 3. Median cell frequencies in the final product are 82% for NK, 16% for γδT and 1% for conventional αβT cells. In addition, feeder cells become undetectable by flow cytometry since day+6 at the start of the expansion.

Representative flow cytometry plots of the cell product before (i.e. Day 0, upper panels) and after (i.e. Day 14) expansion (lower panels) are represented in Figure 1.

The antitumor functions of both NK and γδT cells were assessed and showed that they normally expressed CD107a and produced IFN-γ and TNF-α following in vitro co-culture with target leukemic cell lines K562, 721.221, Daudi, HL60 using different stimulation conditions. Cells were co-cultivated for 4 hours at 1:1 effector:target ratio.

## Claims

1. Process for preparing a cell composition comprising Natural Killer (NK) cells and gamma-delta T (γδT) lymphocytes, said process comprising the steps of:
a) contacting a biological sample comprising NK cells and γδT lymphocytes, isolated from a first subject, with feeder cells treated with a phosphoantigen stimulating agent,
b) contacting said biological sample and said feeder cells from step
a) with a growth medium comprising at least one phosphoantigen stimulating agent and at least one interleukin, for at least 5 days,
c) isolating the cell composition obtained from step b), wherein said cell composition comprises, as a percentage of living cells:
• at least 90% of NK cells and γδT lymphocytes and
• less than 5% of alpha-beta T (αβT) lymphocytes.

2. Process according to claim 1, wherein said sample comprising NK cells and γδT lymphocytes is chosen among: blood, peripheral blood mononuclear cells (PBMCs), tissue sample and iPSC.

3. Process according to claim 1 or 2, wherein said phosphoantigen stimulating agent is chosen in the group consisting of: zoledronic acid, pamidronic acid, HMBPP and BrHPP, and their derivatives.

4. Process according to any of the preceding claims, wherein said interleukin is chosen among: IL-2, IL-21 and IL-15.

5. Process according to any of the preceding claims, wherein in step b) said growth medium comprises zoledronic acid at a concentration of at least 5 µM and human recombinant IL-2 at a concentration of up to 1000 UI/ml.

6. Process according to any of the preceding claims, comprising prior to step a), a step of depletion of said sample from T lymphocytes.

7. Process according to any of the preceding claims, further comprising a step of depletion of said cell composition isolated at step c) from T lymphocytes.

8. Cell composition obtained by a process according to any of claims 1 to 7, said cell population comprising:
• at least 90% of NK cells and γδT lymphocytes and
• less than 5% of αβT lymphocytes.

9. Cell composition according to claim 8, comprising:
• 75 +/-5% of NK cells,
• 25 +/-5% of γδT lymphocytes, and
• less than 1% of αβT lymphocytes.

10. Cell composition according to claim 8 or 9, for its use in medicine.

11. Cell composition for its use in medicine according to claim 10, for the prevention or the treatment of cancer or infection.

12. Cell composition for its use in medicine according to claim 11, for the treatment of cancer.

13. Pharmaceutical composition comprising a cell composition according to any of claims 8 to 9 and a pharmaceutically acceptable carrier.
